# EUROPEAN PATENT SPECIFICATION

(11) **EP 2 708 887 B1**
(45) Date of publication and mention of the grant of the patent: **18.11.2015**
(21) Application number: 13184310.4
(22) Date of filing: 13.09.2013
(51) Int. Cl.: G01N 33/44, G01N 3/18, G01N 11/00

(54) **Apparatus and method for the thermo-mechanical characterisation of materials**
Vorrichtung und Verfahren zur thermisch-mechanischen Charakterisierung von Materialien
Dispositif et procédé de caractérisation thermomécanique de matériaux

(30) Priority: 13.09.2012 GB 201216362
(43) Date of publication of application: 19.03.2014
(73) Proprietor: Reading, Michael, Norwich, Norfolk NR2 3RQ (GB)
(72) Inventor: Reading, Michael, Norwich, Norfolk NR2 3RQ (GB)
(74) Representative: Stephenson, Philip

(56) References cited:
- EP-A2- 0 793 088
- WO-A1-2010/019256
- WO-A2-02/14836
- WO-A2-2012/079076
- DE-C1- 19 754 129
- US-A- 4 426 160
- US-A1- 2012 062 891

## Description

The present invention relates to an apparatus and method for characterizing how the properties of materials change as a function of time and temperature.

### BACKGROUND

There is already an extensive literature on thermal analysis; it is a family of techniques that provide a powerful means for characterizing the properties of materials. These include :-
- Hot stage microscopy including optical microscopy, atomic force microscopy (AFM) and other forms of microscopy that provide images and structural information on a small scale. One method that measures transition temperatures to create an image is called Transition Temperature Microscopy (TTM).
- Dynamic mechanical analysis (DMA)
- Thermomechanical analysis (TMA)
- Differential scanning calorimetry (DSC)

These techniques can provide information on the physical properties of materials including, but not limited to, height, stiffness (also called Modulus), expansion and the enthalpies associated with transitions. All of these techniques determine how the measured property or properties change as a function of time and temperature. An important piece of information that is often obtained using these measurements is the temperature at which transitions occur; examples of transitions of interest include glass transitions and melting points.

In all of these methods the measurement is achieved by one of or a combination of two basic approaches :-
- Locating and/or or tracking directly or indirectly the movement of a sample (often expressed as measuring the movement, displacement, or deformation of the sample) under the action of a force. This is referred to as the Controlled Stress method.
- Directly or indirectly causing the sample to move in a predetermined way (often expressed as controlling the movement, displacement, or deformation of the sample). This is referred to as the Controlled Strain method.

In all cases the force applied to the sample is by means of a component or components in an apparatus that comprises a furnace that heats and cools the sample.

Examples include :-
- When a pointed component is pressed against a point on the surface of a sample using a known force, the degree of penetration (movement) of the component into the sample enables the stiffness of the material at that point to be measured.
- When a force is applied to bend the whole of a sample, the degree of bending (the deformation) enables the stiffness of the sample to be determined.
- When a feedback loop is used to ensure that the degree of bending (the deformation) of a whole sample is a predetermined amount, the magnitude of the force required to achieve this enables the stiffness of the sample to be measured.
- When a sample of a material is heated it expands thereby applying a force to any component that is in contact with the sample surface; when this component is not significantly constrained, the component will move. When this movement is measured as a function of temperature, the thermal expansion coefficient of the material can be measured.
- Calorimeters are often used to measure the enthalpy changes that occur in a sample as a function of temperature; achieving accurate measurements requires that the sample and/or the sample container be accurately positioned in a predetermined place within the calorimeter.

The information these techniques acquire is essential for understanding material properties for a wide range of applications in pure and applied science. The techniques and their benefits are well understood and will not be described in further detail here. An example of a book that reviews the relevant information is Thermal Analysis of Polymers, Fundamentals and Applications, J D Mencel and B R Prime Eds., ISBN 978-0-471-76917-0, John Wiles & Sons (2009).

The various methods of analysis described above require a different instrument for every technique; each instrument uses substantially different transducers and actuators. Consequently there are the following disadvantages:-
- The cost of manufacturing this range of instruments is high.
- More than one instrument is usually required in order to adequately characterize materials; buying several instruments is expensive.
- A great deal of laboratory space is needed to accommodate these instruments.
- There are types of useful analysis that cannot be performed by any of these instruments.

WO02/14836 discloses apparatus and methods for evaluating the rheological properties of Newtonian and non-Newtonian liquids and melts which employs the principle of capillary break-up following rapid stretching of the fluid between two plates.

DE19754129 discloses a measuring system for detecting the contour variation of tensile specimens at various temperatures, the system includes a chamber enclosing a specimin in which strain rods of a test machine protrude, an inspection window and a ventilator for distributing a thermostating medium, a light source with a diffusing plate on the side of the specimen with an inspection tube between the specimen and the inspection window. A camera records specimen images through the window and a translucent flow diverter is positioned between the light source and specimen

US2012/062891 discloses a method for quantitatively determining the viscoelastic properties of a complex medium with suspended particles in suspension. The method includes; a) characterizing the opacity of the medium by measuring the transport length of the medium, b) introducing a sample of the medium into a flask, c) placing the flask in a thermostatically-controlled measuring chamber, d) projecting a coherent light beam on the sample and detecting scattered light in the form of a series of sequenced images, e) analyzing the movement of the particles based on time by calculating the inter-image distance d2, and f) establishing an analytical expression putting into direct relationship for the value of transport length that is determined in stage a), the ratio d2/d2max and the mean square displacement for the purpose of calculating the viscous and elastic moduli of the medium.

EP0793088 discloses a dynamic and thermal mechanical analyzer incorporating a slide driven vertically in an air bearing guidance system with a large displacement capacity, very low friction and low mass. The position of the slide is measured by digitizing and interpolating two quadrature output signals generated by an optical encoder with very high spatial resolution and a long stroke. A force is applied to the slide using a linear permanent magnet motor with high force, high force linearity and low sensitivity to temperature variations. Position signals derived from the digitized and interpolated quadrature output signals are analyzed as a function of the applied force to calculate viscoelastic properties of a sample of material.

US4426160 discloses a method and apparatus for optically making physical measurements of materials which deform upon application of heat thereto. The device comprises a source of light, an at least partly transparent chamber having a heating source which can be programmed to operate as a function of time, and with a porous support designed to support a deformable material within the chamber. A photo-electric receiver and an optical lens are also provided for imaging of the material during deformation on the photo-electric receiver. In order to perform the imaging, the source of light, the porous support, the photo-electric receiver and the optical lens are optically aligned. In a specific application, the apparatus can be used to determine the wetting power of pitches.

### STATEMENTS OF THE INVENTION

To over come these disadvantages the present invention proposes a single basic instrument that is sufficiently versatile to perform the function of multiple instruments as well as providing useful techniques for analysing materials not available with current instruments.

It is to be understood that the term "connected to" may refer to 'a fixed union' or 'being in contact with' or any other type on interaction that means one component is capable of applying a force to another either continuously or discontinuously including, for example, intermittent contact.

It is to be understood that all embodiments of the invention require a "set of components"; sometimes in some situation for convenience, it may be simpler to describe an embodiment by designating some components as subcomponents. It is to be understood that a "set of components' comprises all of the components and subcomponents required to create an embodiment. Where there is a reference to components it is to be understood that this means all components plus all subcomponents.

It is to be understood that a furnace is a chamber that controls the environment the sample experiences including controlling the temperature; this usually means that the temperature can be decreased to be below, and increased to be above ambient temperature.

Reference is made in this specification to the ability of a camera used with suitable software to locate and track the movement of objects within its field of view. It is to be understood that some objects are not capable of being reliably tracked, where "reliably tracked" means that few errors are made when tracking the movement of an object, because the appearance of these objects is not sufficiently distinctive. It is generally the case that objects are more easily located and tracked when these objects are clearly different in appearance from their surroundings.

It is to be understood that the objects that are tracked are called targets.

It is to be understood that references to a camera being used with suitable software are intended to mean that a camera is connected to a computer that is programmed to run the suitable software thereby to analyze the data provided by the camera.

It is to be understood that the term "Cassette" means a device that contains and supports a number of preassembled components.

It is to be understood that all embodiments of the invention can be used in controlled stress mode, controlled strain mode and/or combinations of both.

It is to be understood that the term "primitive heat flux plate" means a conventional heat flux plate of a type well know to one of ordinary skill in the art.

It is to be understood that a composite heat flux plate is a heat flux plate comprising more than one primitive heat flux plate; the combination of the primitive heat flux plates being operable, by means of suitable electrical connections, to function in the same way as a single primitive heat flux plate and also operable, by means of suitable electrical connections, to act as number of primitive heat flux plates connected in series.

In a first aspect of the invention there is provided an apparatus for the thermo-mechanical characterisation of a sample, said apparatus comprising a chamber configured to heat or cool the sample and a set of components located within the chamber, said apparatus including at least one light source and at least one camera situated outside of the chamber, said camera operable to observe one or more of the set of components that are connected to the sample and that apply a force to the sample, characterised in that the location and movement of a component or a plurality of components simultaneously is measured and tracked, including tracking motion that is linear or two dimensional, by analyzing the images obtained from the camera using suitable software.

In a second aspect of the invention there is a method of measuring the tthermo-mechanical properties of a sample (4) as a function of time and temperature comprising placing a sample and a number of components (2,3,7,8,9,10,12) into a chamber configured to heat or cool the sample and the components, one or more of these components being in contact with the sample and applying a force to the same; illuminating the sample with at least one light source; observing a selected component or a plurality of selected components with at least one camera (1); analyzing the images (16, 35,34) obtained with the camera by means of suitable software thereby measuring the location and tracking the movement of the selected components or the plurality of selected components simultaneously thereby to obtain data in respect of the properties of the sample as a function of time and temperature.

Preferably the targets may be arranged to be distinctive in appearance thereby to be reliably located and tracked by the camera when used with suitable software.

Preferably a target that is distinctive in appearance is attached to a component that cannot conveniently made be distinctive in appearance thereby to enable the component that is not distinctive in appearance to be reliably tracked.

Preferably the background behind the targets may be arranged to be different in appearance from the targets thereby creating a contrast between targets and their surroundings thereby making it possible to reliably locate and track the targets by use of the camera in combination with suitable software.

Sets of components may be arranged to be located in Cassettes wherein components are preassembled thereby to be capable of being rapidly loaded into and taken out of a device that comprises all items necessary to institute desired modes of analysis other than the preassembled components, these items to include, but not be limited to, light sources, cameras, suitable software, a furnace and a computer.

One or more of the light sources may be capable of being modulated in intensity under computer control in synchrony with the movement of the sample.

One or more of the light sources may emit colored light. One or more of the light sources may emit ultraviolet light.

One or more of the targets may be fluorescent. One or more of the targets may be luminescent. One or more of the targets may be reflective.

One or more of the set of components may comprise an actuator. One or more of the set of components may comprise a spring. One or more of the set of components may comprise a stylus. One or more of the set of components may comprise a fixed member. One or more of the set of components may comprise a Force transducer. One or more of the set of components may comprise a device for measuring movement. One or more of the set of components may comprise a flat plate. One or more of the set of components may comprise a cylinder. One or more of the set of components may comprise a post. One or more of the set of components may comprise a rotating union. One or more of the components may comprise a clamp. One or more of the set of components may comprise a primitive heat flux plate. One or more of the set of components may comprise a composite heat flux plate. One or more of the set of components may comprise a crucible. One or more of the set of components may comprise any device capable of being used to determine the properties of a material.

### BRIEF DESCRIPTION OF THE DRAWINGS

Figure 1. This is a generalized schematic representation of the invention.
Figure 2. This shows the configuration used in a conventional DMA to cause a shear deformation.
Figure 3. This is a diagram of an embodiment of the invention that enables shear deformation to be applied to a sample.
Figure 4. This shows experimental results acquired using the apparatus illustrated in Figure 3 applied to characterizing the cure of Epoxy glue.
Figure 5. This shows an alternative embodiment of the invention that enables a shear deformation to be applied to a sample.
Figure 6. This shows an embodiment of the invention that enables the cure of films to be characterized.
Figure 7. This Figure shows experimental results obtained using the apparatus illustrated in Figure 6.
Figure 8. This shows the configuration used in a conventional DMA to cause a dual cantilever deformation.
Figure 9. This shows an embodiment of the invention that enables a dual cantilever deformation to be applied to a sample.
Figure 10. This Figure shows experimental results obtained using the apparatus described in Figure 9.
Figure 11. This Figure shows experimental results obtained using the apparatus described in Figure 9 obtained using a more sophisticated approach compared to that shown in Figure 10.
Figure 12. This is a schematic diagram of a conventional Thermomechanical Analyzer.
Figure 13. This shows an embodiment of the invention that acts as a Thermomechanical analyzer.
Figure 14. This Figure shows experimental results obtained using the apparatus shown in Figure 13.
Figure 15. This is a schematic diagram of how Transition Temperature Microscopy works.
Figure 16. The transition temperature measurements at multiple locations shown in this Figure were acquired using the embodiment illustrated in Figure 13.
Figure 17. The profile measurements shown in this Figure were acquired using the embodiment of the invention illustrated in Figure 13.
Figure 18. This shows a diagram of a stylus probe that has more functionality than that shown in Figure 13.
Figure 19. A composite heat flux plate is shown that can accommodate crucibles of widely different sizes.
Figure 20. This illustrates how, by making an electrical connection between two of the heat flux plates, a single heat flux plate is formed.
Figure 21. This illustrates how a composite heat flux plate is formed by connecting the individual heat flux plates in series.
Figure 22. This shows how a composite heat flux plate can accommodate a large crucible.
Figure 23. This illustrates how crucibles can be located accurately on a heat flux plate.
Figure 24. This illustrates how components can be preassembled to create Cassettes.

### DESCRIPTION OF INVENTION

The present invention comprises a device and methods that overcome the limitations of existing approaches to characterizing the properties of materials. We consider the existing approaches in turn and describe how embodiments of the current invention can provide superior performance in a more cost effective way. Also described are embodiments of the invention that offer useful modes of measurement that current instruments cannot provide.

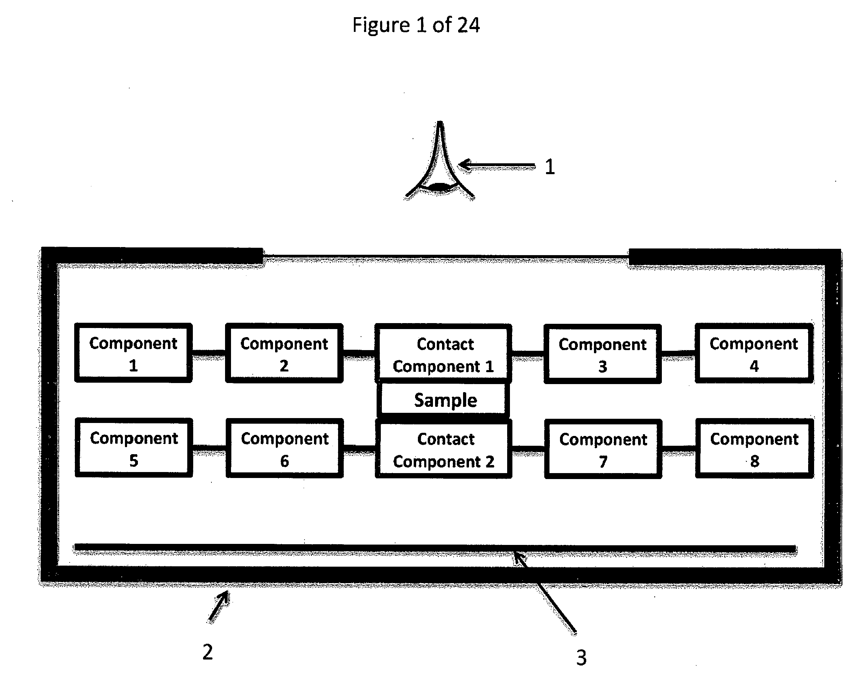

A) In Figure 1 a generalized schematic diagram of the invention is provided that has the following features : -
   1. A camera observes a sample or part of a sample and/or one or more of a set of components. By use of the camera used with suitable software the location of the sample and/or part of the sample and/or selected components are determined and their movements are tracked; these selected components are called targets. The sample can also be selected to be a target.
   2. Some components are designed to be distinctive in appearance so that the camera used with suitable software can reliably locate and track their movements. In some cases, being located and tracked is the only function a target has; such targets may be attached to components whose movements need to be tracked but that cannot be reliably tracked because they are not intrinsically sufficiently distinctive in their appearance. Targets of distinctive appearance of this type are not explicitly shown in Figure 1; any of components 1 to 8 may have a target of this type attached to them as required.
   3. The appearance of the background behind the components can be controlled in order to make it easier to locate and track targets.
   4. The components are connected to each other and to the sample in various configurations depending upon the type of measurement being made.
   5. The contact components are in contact with the sample
   6. In some embodiments there are more than two contact components (when this is the case contact components 1 and 2 in the diagram are divided so that there is contact component 1a, contact component 1b etc.).
   7. The sample is located in a furnace that enables its environment to be controlled; typically the temperature of the sample can be controlled so that the sample can be heated and cooled. The furnace contains only the sample, or the sample with one or more of the components.
   8. By analyzing the data that are obtained about the location and movement of the targets important properties of the sample are determined.
   9. Some components may be transducers that measure quantities such as force, movement and temperature; these may supply supplementary information that is used to assist in determining the properties of the sample.

Figure 1 is a simplified representation of the invention. The number of components can exceed eight if required. In some cases it may be convenient to designate some components as subcomponents to simplify the schematic representation of some embodiments. In all cases a set of components comprises all components plus all subcomponents.

A feature of the invention is that different sets of components are sometimes used for different modes of analysis for example a Scratch Test requires a Stylus while a shear cell requires two flat plates. These sets of components can be changed while the light sources, the cameras, the furnace, the computer and all associated control devices remain unchanged. This enhances the versatility of the invention. Having acquired the basic equipment different modes can be accessed simply by using relatively inexpensive Cassettes that comprise the preassembled components necessary for a particular mode of analysis. These cassettes can then be rapidly inserted into and removed from the basic instrument.

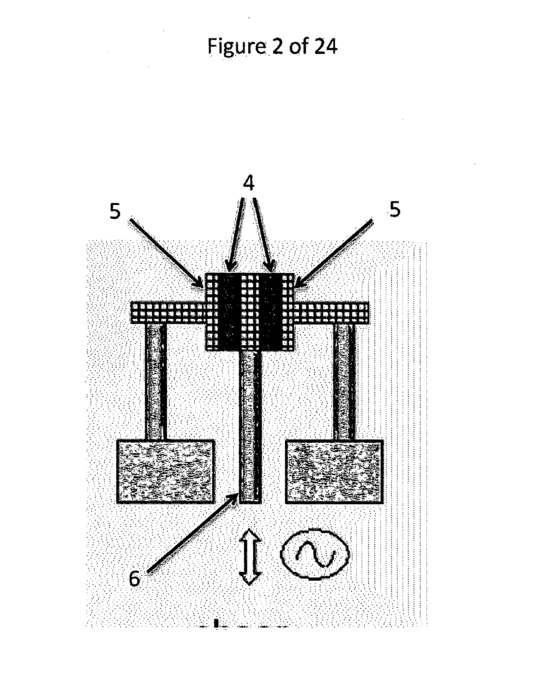

B) Figure 2 shows the configuration used in a conventional DMA to cause a shear deformation. The sample 4 is clamped onto both sides of an actuator 6 that moves up and down while the other faces of the sample are clamped using member 5 so that their movement is constrained; more details can be obtained by reference to the textbook cited above or other similar documents.

The disadvantages of a conventional DMA shear cell are :-
- The sample must be clamped and this introduces a poorly controlled variable that can be different for different experiments.
- Low viscosity liquids cannot be characterized.
- If the sample adheres to the clamps it can be expensive to rescue them for future use.
- If the sample flows out of the clamps it can damage the apparatus.

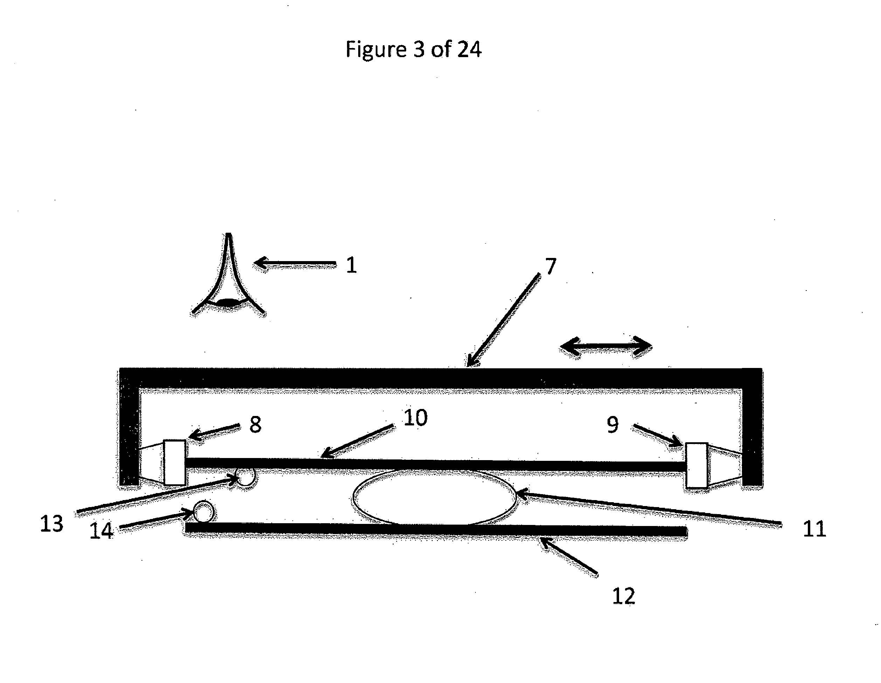

Figure 3 shows a diagram of an embodiment of the invention that enables shear deformation to be applied to a sample. One of the ends of each of the two cantilever springs 8 and 9 are linked together by member 7; this member is moved from left to right by an actuator (not shown). The other ends of springs 8 and 9 are in contact with contact component 1 (numbered 10 in the Figure), which is a flat plate, for example a glass microscope slide. The action of springs 8 and 9 when member 7 moves transmits a force through the springs that acts on 10 causing it to move from left to right. The movement of contact component 2 (numbered 12 in the Figure) is constrained so that it can move only a little in response to any force transmitted through the sample 11. Contact component 2 (numbered 12 in the Figure) is a flat plate, for example a glass microscope slide. The movements of contact components 1 and 2 (numbered 10 and 12 in the Figure) are followed by means of camera 1 observing the two targets 13 and 14 attached to 10 and 12. Contact components 1 and 2 (numbered 10 and 12 in the Figure) can be made of any substantially rigid material provided allowance is made by the provision of holes other means so that the targets 13 and 14 are visible to the camera 1. The whole except the camera 1 is placed in a furnace (not shown).

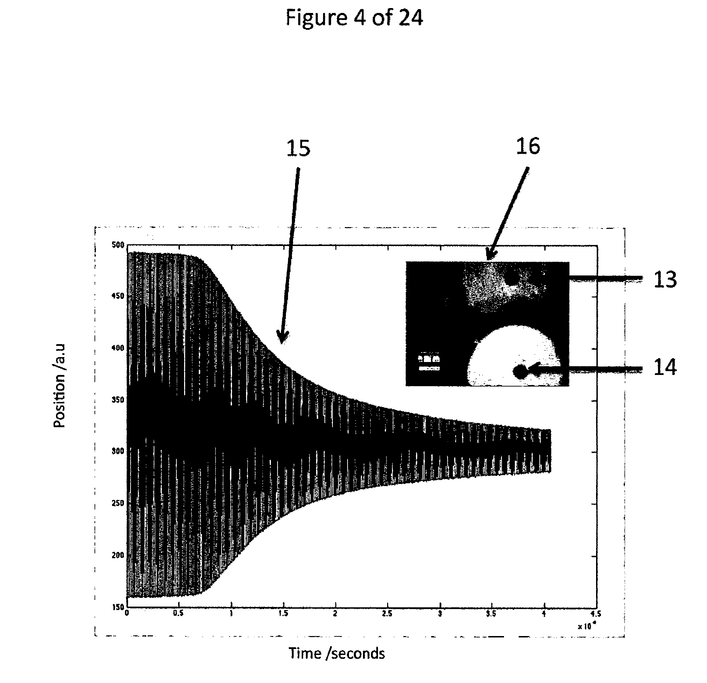

Figure 4 shows experimental results acquired using the apparatus illustrated in Figure 3 applied to characterizing the cure of Epoxy glue. Targets 13 and 14 are shown on photograph 16 taken with the camera. These targets were attached to contact components 1 and 2, in this case both glass microscope slides. The camera used in conjunction with suitable software tracked the movements of the targets. The difference between the targets gives a measure of the shear strain experienced by the sample 11 while the amount of deflection of the Cantilever Springs provides a measure of the force. The graph 16 shows the amplitude of the distance between the top and bottom slides and how it changes with time because the glue becomes more viscous.

This embodiment of the invention has the following advantages over the conventional method :-
- The use of simple springs and an inexpensive camera means this apparatus can be built more cheaply that a conventional DMA.
- No clamping is required thus variability between experiments is much reduced.
- Because there is no need to clamp the sample the experiment is much simpler to perform.
- Low viscosity fluids can be used as samples.
- If the sample adheres to the glass microscope slides they can be disposed of as they are inexpensive.
- The sample is contained between the two glass microscope slides and cannot flow out and so damage the apparatus.

With reference to Figure 1 the apparatus shown in Figure 3 can be described by designating :-
- Components 1 and 4 are connected (together the 2 components are represented by 7); they are moved by an actuator (not shown).
- Component 2 is a Cantilever Spring (8).
- Component 3 is a Cantilever Spring (9).
- Contact component 1 is a flat plate (10).
- The sample is the sample (11).
- Contact component 2 is also a flat plate (12); its position is substantially fixed.
- There are two targets one on the top flat plate (13) and one on the bottom (14); these are tracked by the camera (1).
- Components 5,6,7 and 8 are not present.

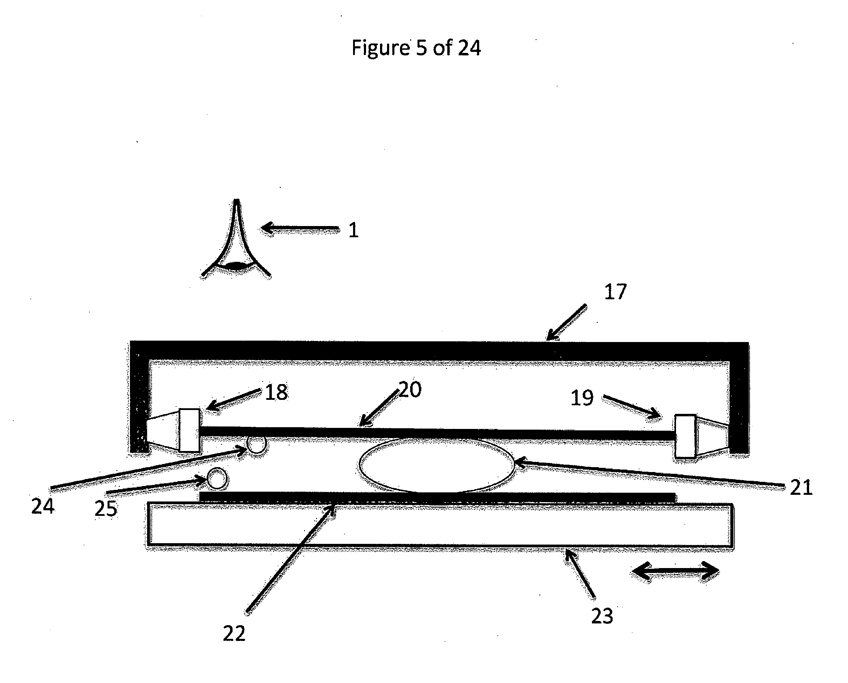

An alternative to the configuration shown in Figure 3 is that illustrated in Figure 5. This shows another embodiment of the invention that enables a shear deformation to be applied to a sample. The bottom surface 23 is in contact with contact component 2 (numbered 22 in the Figure); 22 is a flat plate, for example a glass microscope slide. Bottom surface 23 is an actuator that moves contact component 2 from side to side; this causes contact component 2 (numbered 22 in the Figure) to move from side to side. This transmits a force through sample 21 to contact component 1 (numbered 20 in the Figure); 20 is a flat plate, for example a glass microscope slide. This force causes the contact component 1 to apply pressure on the free (unfixed) ends of the cantilever springs 18 and 19, the other ends are attached to the fixed member 17. The force causes contact component 1 (numbered 20 in the Figure) to move. The movements of contact components 1 and 2 are followed by means of the camera 1 observing the two targets 24 and 25 attached to 20 and 22. Contact components 1 and 2 can be made of any substantially rigid material provided allowance is made by the provision of holes other means so that the targets 22 and 23 are visible to the camera 1. The whole, except the camera, is placed within a furnace (not shown).

Figure 5 can be described with reference to Figure 1 by designating :-
- Components 1 and 4 are fixed members (they are shown as connected to each other in Figure 5 thus the 2 components are represented by 17)
- Component 2 is a Cantilever Spring (18).
- Component 3 is a Cantilever Spring (19).
- Contact component 1 is a flat plate (20).
- The sample is the sample.
- Contact component 2 is a flat plate (22)
- Component 6 in an actuator (23) that moves contact component 2 (22).
- There are two targets, one on the top flat plate (24) and one on the bottom (25); these are tracked by the camera (1).
- Components 5,7 and 8 are not present.

The difference between the configuration shown in Figure 3 and that shown in Figure 5 is that in the former one end of the Spring is attached to an actuator and both ends of the spring move, in the latter one end of the spring is held in a fixed position. Both of these configurations can be used to measure the viscosity and other properties of materials and how these properties change with time and temperature. In general all of the embodiments that employ springs or similar components have the option of connecting an actuator to one end of the spring(s) or keeping one end of the Spring(s) fixed while using an actuator to move another component in the apparatus.
C) Some coating systems that cure, such as some types of paint and some types of glue, require an open surface exposed to the air in order to change from a liquid to a solid. This type of cure cannot be properly characterized using current DMA's.

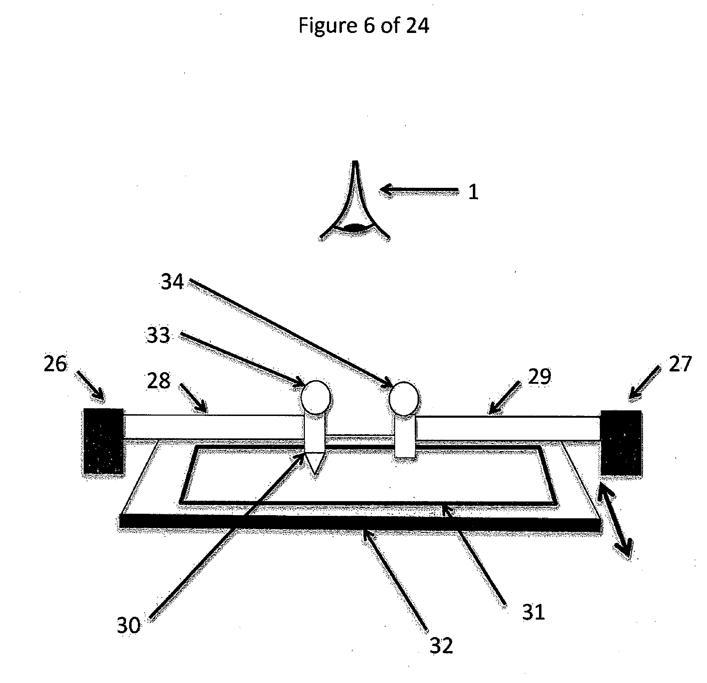

In Figure 6 the film 31 has one surface open to the atmosphere; some coatings need to lose solvent or have access to the chemical species available in the air in order to cure. The sample 31 is located on contact component 2 (numbered 32 in the Figure), which is a flat plate, for example a glass microscope slide. Contact component 2 is moved forwards and backwards by an actuator (not shown). A Stylus 30 is contact component 1; it is attached to the free end of a cantilever spring 28; the other end is held in a fixed position by member 26. The Stylus tip penetrates the sample 31. As the sample 31 becomes more viscous the force applied to the Stylus 30 by the movement off flat plate 32, increases and this causes the Stylus 30 to move by increasing amounts. This movement is followed by camera 1 used with suitable software that together track the movement of a target 33. There is a reference target 34 held in position by members 29 and 27 that are substantially rigid. Contact component 2 can be made of any substantially rigid material. The whole except the camera 1 is placed in a furnace (not shown).

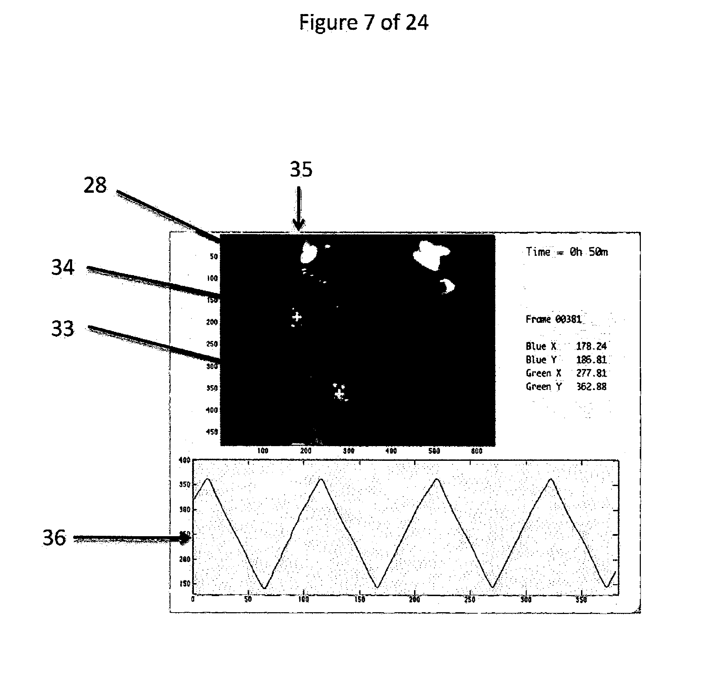

Figure 7 shows experimental results obtained using the apparatus illustrated in Figure 6. The targets 33 and 34 and the cantilever spring 28 are indicated on photograph 35 taken with camera. Graph 36 provides experimental data that show that the target 34 is moved by the action of contact component 2. This is because the coating is substantially cured.

This embodiment of the invention has the following advantages over the conventional method :-
- It provides a method of measuring the cure of films that require one surface to be exposed to the atmosphere that is not available with conventional instruments.
- It provides a measure of how the viscosity of the film increase, how the film can be scratched when soft and how resistant the film is to penetration as it becomes hard; these measurement are not available to conventional Thermal Analysis instruments.

Figure 6 can be described with reference to Figure 1 by designating :-
- Component 1 is a fixed member (26).
- Component 2 is a Cantilever Spring (28)
- Contact component 1 is Stylus (30).
- The sample is the sample (31).
- Contact component 2 is a flat plate (32) that is moved by an actuator (not shown)
- There is a target (33) attached to the top of the Stylus.
- There is a reference component shown in Figures 6 and 7 that is not represented in the Schematic shown in Figure 1 for the sake of simplicity.
- Components 3,4,5, 6, 7 and 8 are not present.

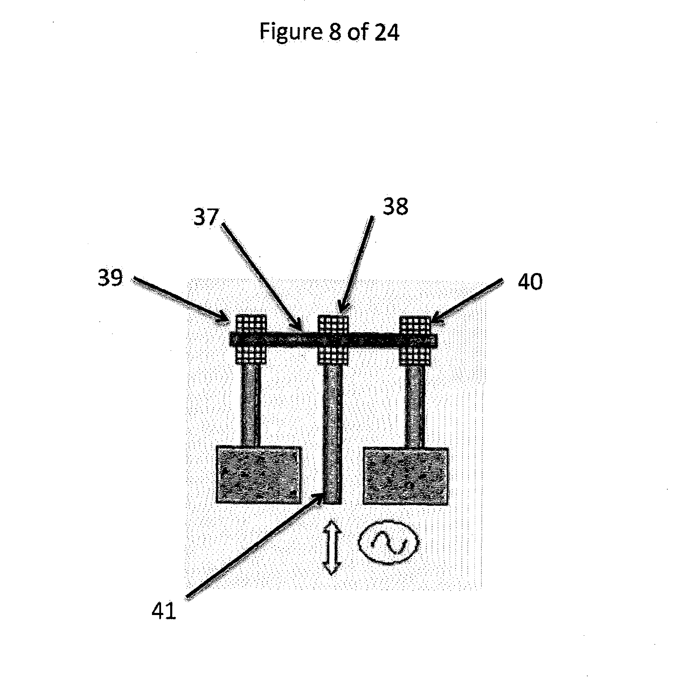

D) A frequently used method employed by conventional DMA's is to deform a sample is a dual cantilever mode, this is illustrated in Figure 8. The two ends of sample 37 are held in a fixed position by clamps 39 and 40. The actuator 41 is attached to the middle of the sample by clamp 38. The actuator moves up and down thereby deforming the sample. More details can be obtained by reference to the textbook cited above or other similar documents.

The disadvantages of a conventional DMA dual cantilever mode are :-
- The sample must be clamped and this introduces a poorly controlled variable that can be different for different experiments.
- The clamps constrain the bending profile of the sample in ways that introduce errors when calculating the stiffness of the sample.
- If the sample adheres to the clamps it can be expensive to rescue them for future use.
- If the sample flows out of the clamps it can damage the apparatus.

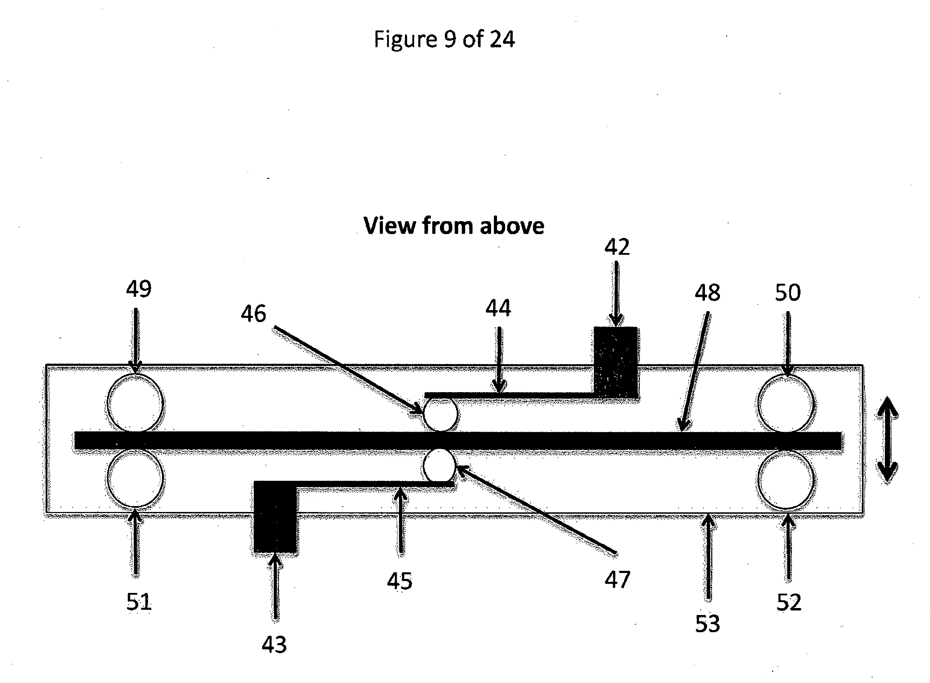

Figure 9 shows an embodiment of the invention that enables a dual cantilever deformation to be applied to a sample. The apparatus consists of contact component 1 that impinges on the sample 48 and causes it to deform. In this case contact component 1 comprises platform 53 on which are located four posts 49, 50, 51 and 52; these are in contact with the two ends of the sample 48. Contact component 1 is connected to an actuator (not shown) that causes it to oscillate in the plane orthogonal to the long axis of the sample 48. The centre of the sample 48 is held by two Cantilever Springs 44 and 45 by means of two cylinders connected to the ends of the springs; these are located beneath targets 46 and 47 (and so are not visible in the Diagram); the cylinders enable a blunt contact to be made with the sample 48 so it is not damaged. The targets are located and their movement tracked by a camera used with suitable software. The other ends of the springs are held in fixed positions by members 42 and 43. As the bottom platform 53 moves a force is exerted by the posts 49, 50, 51 and 52 acting against the ends of the sample 48. This force is transmitted to the Cantilever Springs 44 and 45 causing the targets 46 and 47 to move. The stiffer the sample, the greater is the movement of the targets. All components except the springs 44 and 45 and the sample 48 can be made of any substantially rigid material. The whole except the camera is placed in a furnace (not shown) that enables the environment experienced by the sample to be controlled, for example its temperature can be controlled.

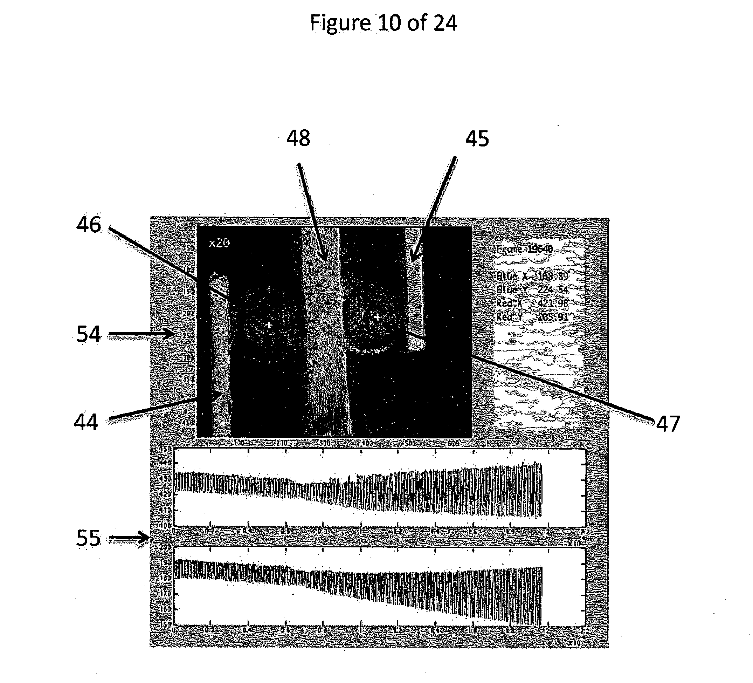

Figure 10 shows experimental results obtained using the apparatus described in Figure 9 while the sample 48 is cooled. Targets 46 and 47 and the Cantilever Springs 44 and 45 are indicated in photograph 54 taken by the camera. The graphs 55 provide experimental data that show that the amplitude of the motion of the two targets 46 and 47 becomes greater as the sample is cooled; this is because the sample gets stiffer.

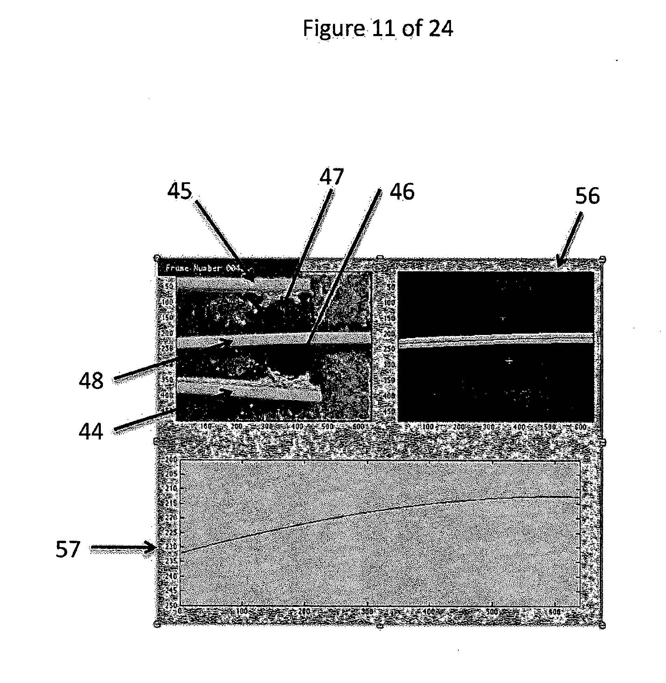

Figure 11 shows experimental results obtained using the apparatus described in Figure 9. It illustrates an alternative analysis to that shown in Figure 10. The targets 46 and 47, the Spring Cantilevers 44 and 45 and the sample 48 are all indicated on the photograph taken by the camera. In this case the springs 44 and 45 and the sample 48 also become targets; their deformation is tracked as shown in the schematic representation 56. A mathematical expression is fitted to model the behavior of the sample and this is shown in the graph 57.

These embodiments of the invention have the following advantages over the conventional method:-
- The use of simple springs and an inexpensive camera means this apparatus can be built more cheaply than a conventional DMA.
- No clamping is required and variability between experiments is much reduced.
- Because there is no need to clamp the sample the experiment is much simpler to perform.
- If the sample adheres to parts of the apparatus replacing them is not expensive.
- The option of using the Spring Cantilevers and the sample as targets and modeling their behavior with mathematical expressions permits a much more sophisticated analysis than is possible using conventional DMA, which only provides 2 data, a force and a position.

The version of Figure 9 shown in Figure 10 can be represented with reference to Figure 1 by designating :-
- Component one is a fixed member (42).
- Component 2 is a Cantilever Spring (44).
- Contact component 1a is a cylinder that provides for a blunt contact with the sample that minimizes the chances of the sample being damaged, attached to it is a target (46), contact component ta is not shown because it is concealed beneath target 46.
- Component 4 is a fixed member (43).
- Component 3 is a Cantilever Spring (45).
- Contact component 1b is a cylinder that provides for a blunt contact with the sample that minimizes the chances of the sample being damaged, attached to it is a target (47), contact component 1b is not shown because it is concealed beneath target 47.
- The sample is the sample (48).
- Contact component 2 is a platform (53) with 2 pillars at each end into which the ends of the sample are inserted (49, 50, 51 and 52).
- Component 6 and/or component 7 are actuators that move contact component 2.
- Component 5 and 8 are not present.

The version of Figure 9 shown in Figure 11 can be represented with reference to Figure 1 by designating :-
- Component 1 is a fixed member (42).
- Component 2 is a Cantilever Spring and is a target (44).
- Contact component 1a is a cylinder that provides for a blunt contact with the sample that minimizes the chances of the sample being damaged, attached to it is a target (46), contact component 1a is not shown because it is concealed beneath target 46.
- Component 4 is a fixed member (43)
- Component 3 is a Cantilever Spring and is a target (45).
- Contact component 1 b is a cylinder that provides for a blunt contact with the sample that minimizes the chances of the sample being damaged, attached to it is a target (47), contact component 1 b is not shown because it is concealed beneath target 47.
- The sample is the sample and is a target (48).
- Contact component 2 is a platform (53) with 2 pillars at each end into which the ends of the sample are inserted (49, 50, 51 and 52).
- Component 6 and/or component 7 are actuators that move contact component 2.
- Components 5 and 8 are not present.

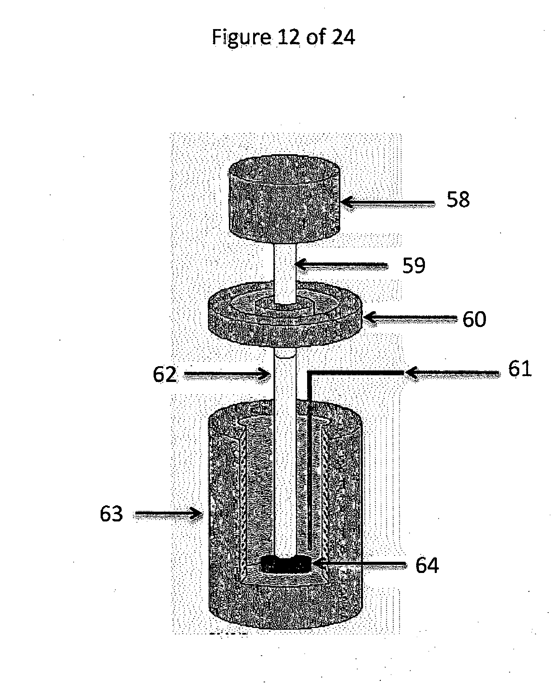

E) A popular conventional method is Thermomchnical Analysis (TMA); a schematic diagram of a TMA is shown in Figure 12. A force is exerted by force motor 58 through a drive shaft 59 that is connected to a probe 62. The probe 62 transmits the force to a sample 64 in a furnace 63 that can be heated and cooled. The position of the drive shaft is measured by position sensor 52. Typically, as the temperature of the sample 64 is increased it will soften and the probe 62 will penetrate into it; this movement is measured by motion sensor 60. In this way the temperature of a transition that causes softening is measured; more details can be obtained by reference to the textbook cited above or other similar documents.

The disadvantages of conventional TMA are :-
• The instrument is large and expensive.
• Heating rates are slow.
• Only one point on a sample is analyzed.
• The contact area of the probe is large

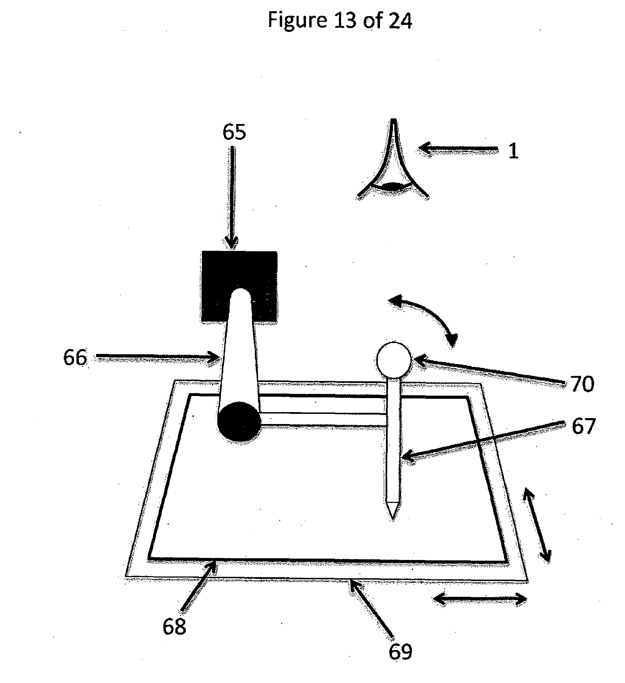

Figure 13 shows an embodiment of the invention that acts as a thermomechanical analyzer. The apparatus consists of contact component 1 which is Stylus 67 so arranged as to be able to rotate about a rod 66 that is attached to a support 65 that can be stationary or can be moveable so as to raise and lower the Stylus 67. The force applied by the tip of the Stylus to the surface of the sample 68 can be varied by adding weights to the Stylus or by means of a magnetic field or by means of an actuator (not shown). The sample 68 is located on contact component 2, which is platform 69 that can, in some embodiments of the invention be moved in two dimensions. When the sample 68 is heated, if it softens the tip of the Stylus 67 penetrates the sample 68 and the Stylus 67 rotates so causing the target 70 to move. The motion of this target is tracked by camera 1 used with suitable software. This then enables a softening transition to be measured. All components except the sample can be made of any substantially rigid material. The whole except the camera is placed in a furnace (not shown).

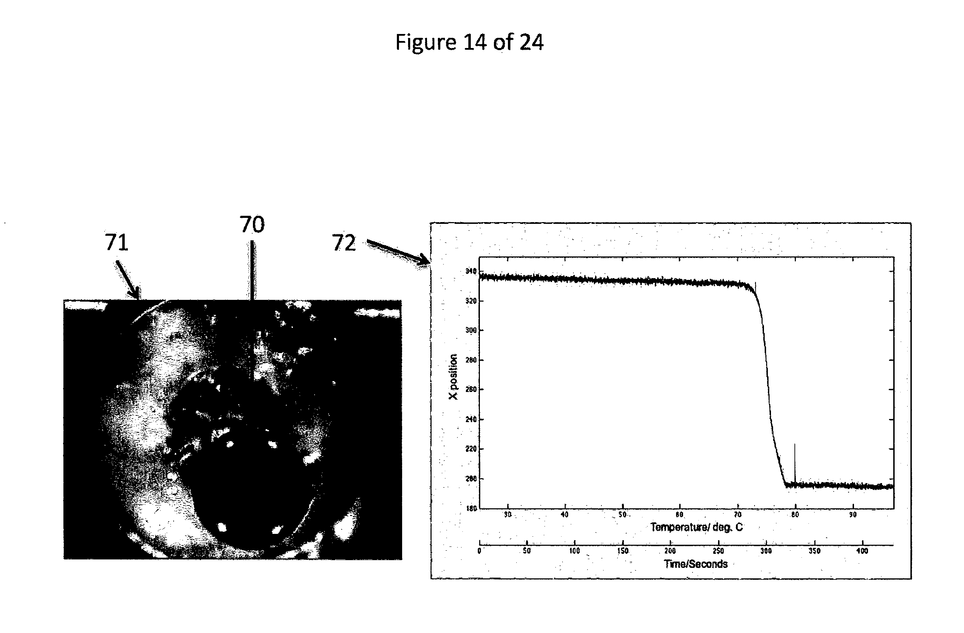

Figure 14 shows experimental results obtained using the apparatus shown in Figure 13. The target 70 is indicated on the photograph 71 taken by the camera. The Stylus 67 is not visible because it is located beneath the target 70. The point of the Stylus was resting on the surface of a polymer that melted at 73°C, the polymer was located in a furnace that controlled the sample temperature. The temperature was increased linearly with time at a rate of 10°C/minute and at 73°C the Stylus penetrated the sample so the target 70 moved forward as the Stylus rotated. This movement was followed by the camera used with appropriate software and this is recorded in the graph 72; from this the softening temperature was determined.

This embodiment of the invention has the following advantages over the conventional method :-
- The use of an inexpensive camera to following the motions of components means this apparatus can be built more cheaply than a conventional TMA.
- Because the apparatus uses a mechanism and a furnace that is much smaller than the conventional instrument, heating rates can be much faster and so experiments can be done much more quickly.
- Because the stylus is much smaller than the probe used in the conventional apparatus, tips with smaller contact areas can be used this means smaller regions can be analyzed.

Figure 13 can be represented with reference to Figure 1 by designating :-
- Component 1 is a member (65) that is fixed during the experiment but can be used to raise the Stylus between experiments.
- Component 2 is a member (66) one end of which can rotate.
- Contact component 1 is a stylus (67).
- The sample is the sample (68).
- Contact component 2 is a flat plate (69).
- Component 6 and component 7 are actuators (not shown) that move contact Component 2 in two dimensions.
- There is a target (70) attached to contact component 1.
- Components 3, 4, 5 and 8 are not present.

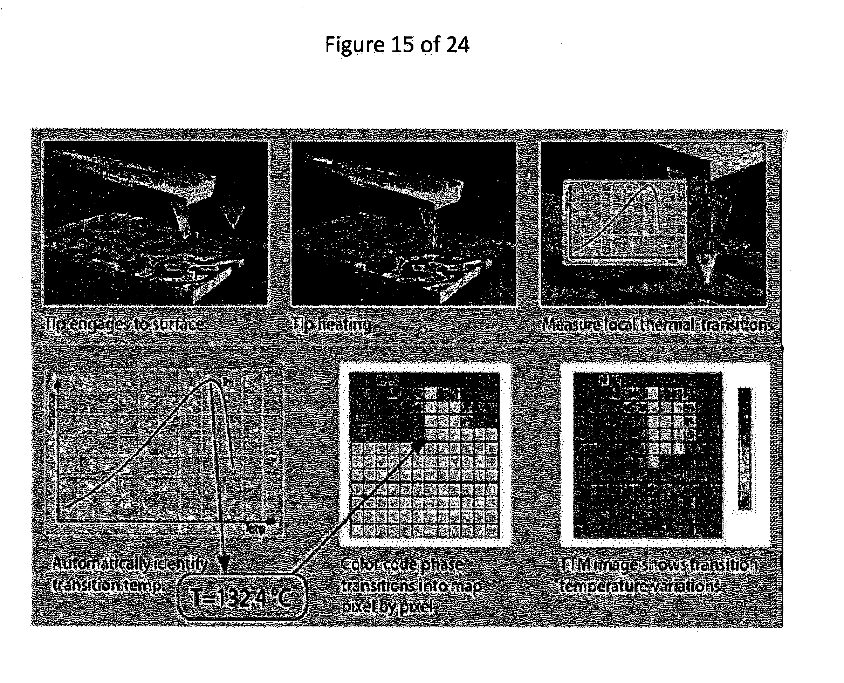

F) Images of samples can be obtained that are based on the temperature at which a transition occurs on a surface; the conventional approach to doing this is called Transition Temperature Microscopy (TTM). Figure 15 provides a schematic diagram of how Transition Temperature Microscopy works. A probe that can be heated is placed on a location on the surface of a sample surface. It is heated until a transition is detected. The temperature at which this transition occurred is determined automatically and this measurement is used as a value for a pixel in an image. More details can be found at :http:/twww.bruker-axs.com/atomic_force_microscopy_webinar_archives.html

The disadvantages of conventional TTM are :-
- The tip is heated and this can change adjacent material so that subsequent measurements on the changed material give an inaccurate picture of the structure of the sample.
- Because the probe is fragile it must be placed on the surface carefully to avoid damaging it, this means images are acquired slowly.
- The heated probes are expensive.
- Cooling the sample well below ambient is currently not possible and this is necessary to study a broad range of samples.

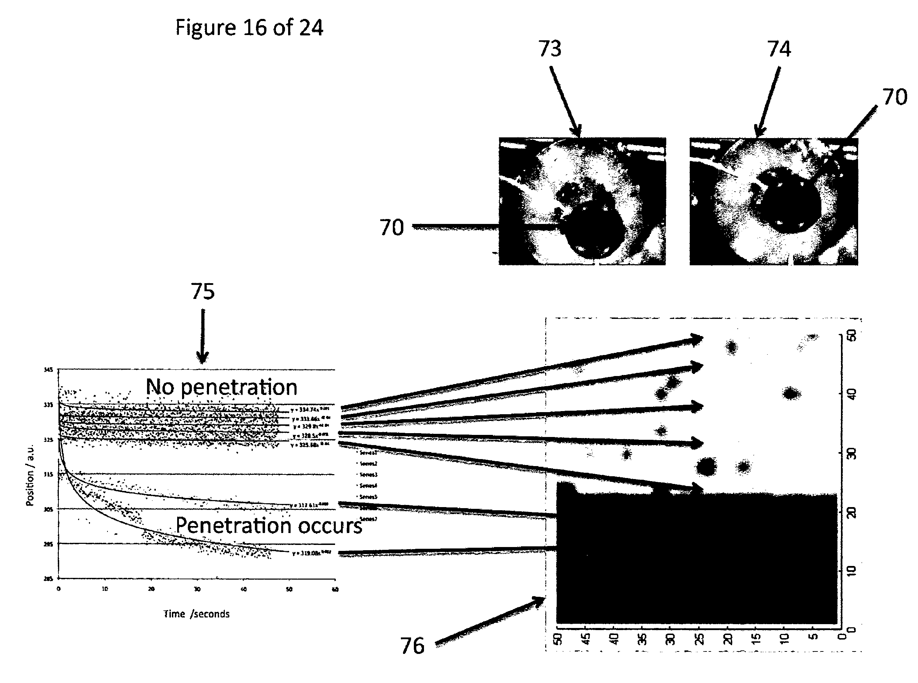

Figure 16 shows results that were acquired using the embodiment illustrated in Figure 13 in the following way: the Stylus (not visible because it is located beneath the target 70) was placed at a location on the sample surface; the position of the target 70 was monitored, if it moved over time then the material under the tip was soft if it did not move then the material at that point was hard. During this period the sample was held at an isothermal temperature. After a period of time the Stylus was lifted and placed at another location. This procedure was repeated a number of times then the temperature of the sample was increased to another isothermal temperature. When the material under the tip of the Stylus went from being hard to being soft as the temperature was increased then a transition had occurred. The process of relocating the Stylus was then repeated. Photograph 73 and 74 show the positions of target 70 after a period of time had elapsed at two different locations at the same temperature. In photograph 74 the target 70 is further forward than in photograph 73. This is because the Stylus penetrated the sample in photograph 74 but not in photograph 73. The results for measurements at six locations at one temperature are shown in the graph 75. At two of the points the tip penetrated over time thus the material was soft and so it had gone through a transition. The positions of the measurements are indicated in image 76, it can be seen that the two points where a transition occurred are located on the dark material. In this way transition temperatures were mapped.

This embodiment of the invention has the following advantages over the conventional method :-
- The changes at each location can be followed as a function of temperature; the thermal history of each , location is unambiguously known and is the same everywhere on the sample. This means the potential for false results that exists with the conventional approach are eliminated.
- The probe does not have to be fragile, a wide range of robust styluses can be used, this means measurements can be made more quickly.
- The Stylus need not be expensive.
- It is easy to cool the sample well below ambient.

The correspondence between the configuration used for this type of measurement and Figure 1 is the same as that for Figure 13 given above.
G) A commonly used technique for characterizing surfaces is Profilometery, a method of acquiring an image or part of the topography of the surface of a sample. In this method a stylus is moved over a surface and the deflections upwards and downwards measure the heights of features on the surface. This type of technique can be used with a stage that heats and cools the sample.

The disadvantages of conventional Profilometery are :-
- These are dedicated instruments that perform only one function.
- Changing the sample temperature over a wide range is not possible.
- Cooling the sample is not possible.

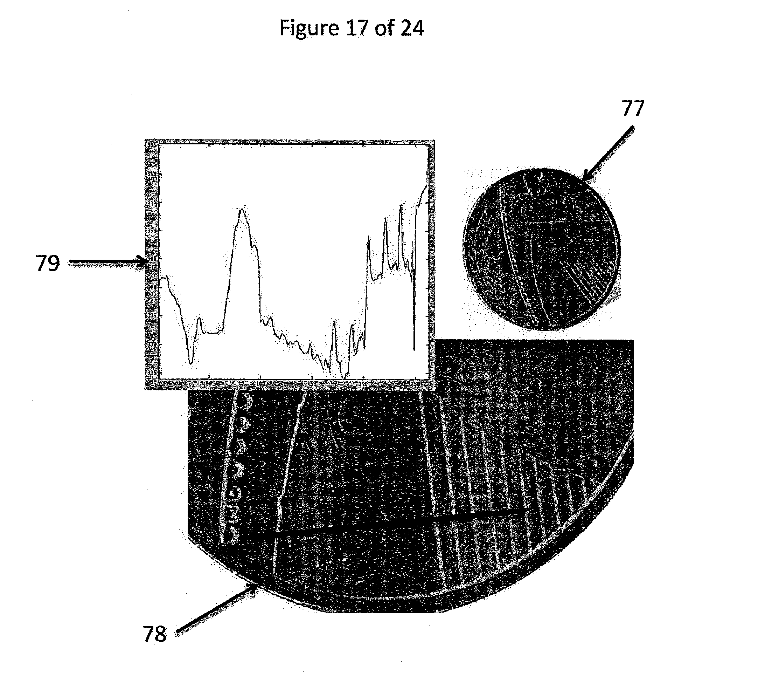

The measurements shown in Figure 17 were acquired using the embodiment of the invention illustrated in Figure 13. The Stylus was placed on the surface of the coin 77. The path over the coin of the Stylus is shown as a black line on an enlarged part of the photograph of the coin 78. The coin was moved while the Stylus was still in contact with it. The Stylus moved up and down as it encountered raised features and dips. The motions of the target were tracked using the camera used with suitable software and from this information a profile was constructed; this is shown in graph 79.

This embodiment of the invention has the following advantages over the conventional method :-
- This embodiment of the invention can be used together with the other embodiments at very little extra cost.
- The temperature of the sample can be varied over a wide temperature range.

The correspondence between the configuration used for this type of measurement and Figure 1 is the same as that for Figure 13 given above.

H) The embodiments C, E, F and G show an advantage of the present invention; these different methods all use a Stylus as contact component 1 while contact component 2 is a flat plate that can move in two dimensions. Thus:-
- This ability to achieve many methods of analysis with a single configuration means the invention is versatile.
- An experimenter can more easily, conveniently and cost-effectively obtain a broad range of material properties compared to the conventional instruments.
- The conventional approach requires many different instruments; even if these instruments are available to the experimenter, the present invention offers modes of analysis not possible with conventional instruments.

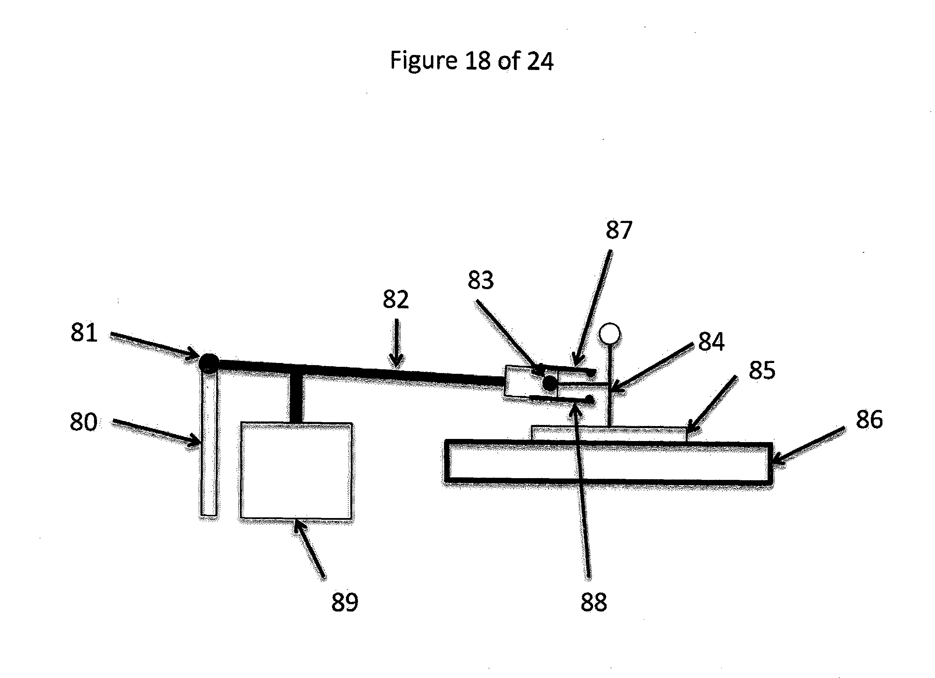

The greater versatility of the present invention compared to current instruments can be further enhanced by use of a more sophisticated stylus Probe. Figure 18 shows a diagram of a stylus probe that has more functionality than that shown in Figure 13. One end of the rod 82 is attached to fixed member 80 about which it can rotate. Stepper motor 89 can be used to raise and lower the rod 82. The Stylus 84 (contact component 1) can rotate about union 83 but this rotation is restricted by springs 87 and 88. When the Stylus 84 is not touched by either spring 87 or 88 then it can move up and down freely thus it can act as a profilometer as demonstrated in Figure 17. When the rod 82 is set at an angle at which spring 87 presses down on Stylus 84, a controlled downward force can be applied that depends on the degree of bending of spring 87. This can be used to perform Thermomechanical Analysis as illustrated in Figure 14 and Transition Temperature Mapping as illustrated in Figure 16 with the additional advantage that the force applied by the Stylus can be varied under computer control. In the same way, spring 88 can contact the Stylus 84 and apply an upward force. A significant upward force is required to raise the Stylus when it adheres to the surface of the sample 85. Sample 85 is located on contact component 2 (numbered 86 in the Figure) which can be moved in the same way as contact component 2 in Figure 13, i.e. in two dimensions. As an alternative to a rotating Stylus, the rotating union can be dispensed with and a spring connected to rod 82 can be used to support the Stylus. This spring would have a high enough spring constant to substantially support the weight of the Stylus but a lower spring constant than spring 87 and 88.

Figure 18 can be represented with reference to Figure 1 in a similar way to Figure 13; the main difference being that component 2 has the subcomponents :-
- A rotating union (83).
- An upper spring (87).
- A lower spring (88).

I) Differential Scanning Calorimeters (DSC) are widely used to characterize a variety of material properties. For them to function well the crucibles must be positioned accurately on the instrument's sensors. In the most commonly used form of DSC, this sensor is a heat flux plate comprising two thermocouple junctions connected in opposition so the output measures the temperature difference between a sample and a reference position. Robot devices are used, called auto samplers, have an arm that picks up a crucible and places it on the heat flux plate; more details can be obtained by reference to the text book cited above or other similar documents.

The disadvantages of conventional methods of placing a crucible in a DSC are :-
- The robots used are expensive and comprise many actuators and moving parts.
- They do not track the movement of the crucible as it is placed on the DSC and then verify by automated visual inspection that it has been placed in the correct position.
- They are not able to move and place correctly a wide range of crucible sizes within a complex composite heat flux plate.

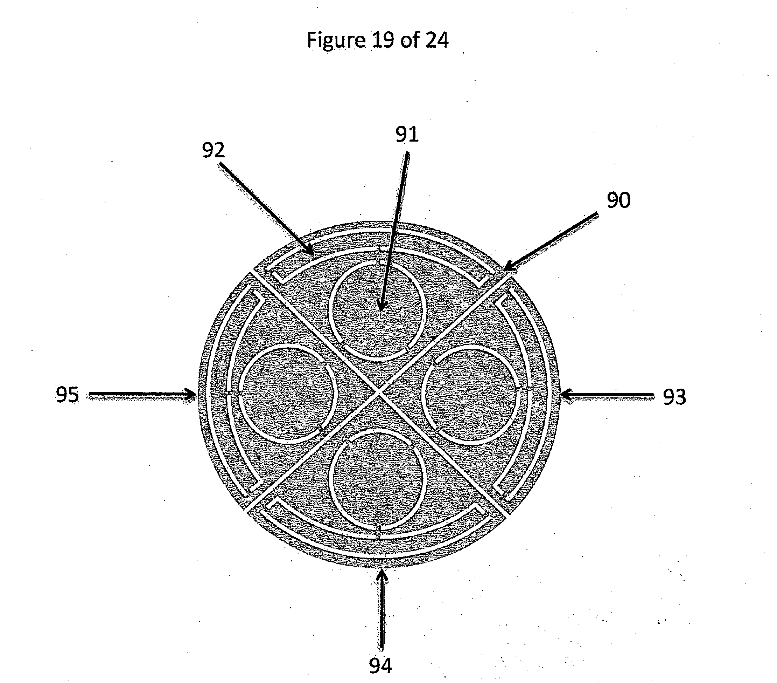

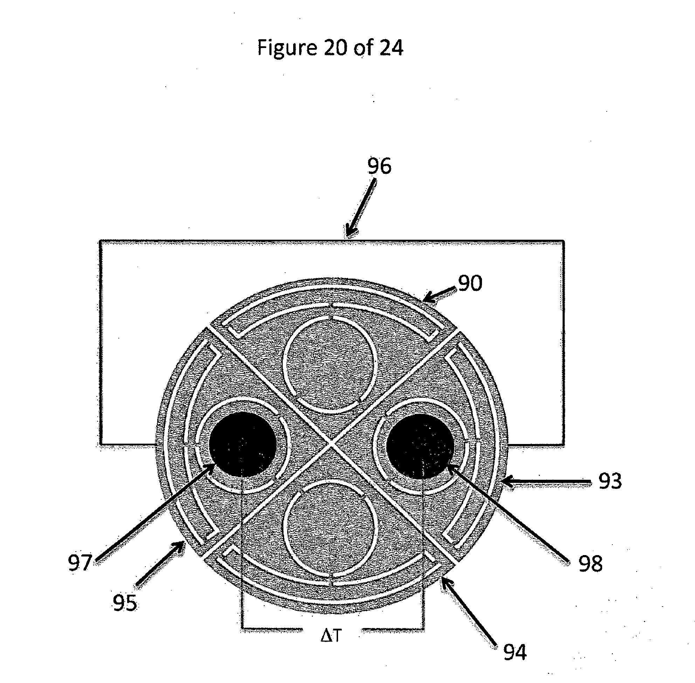

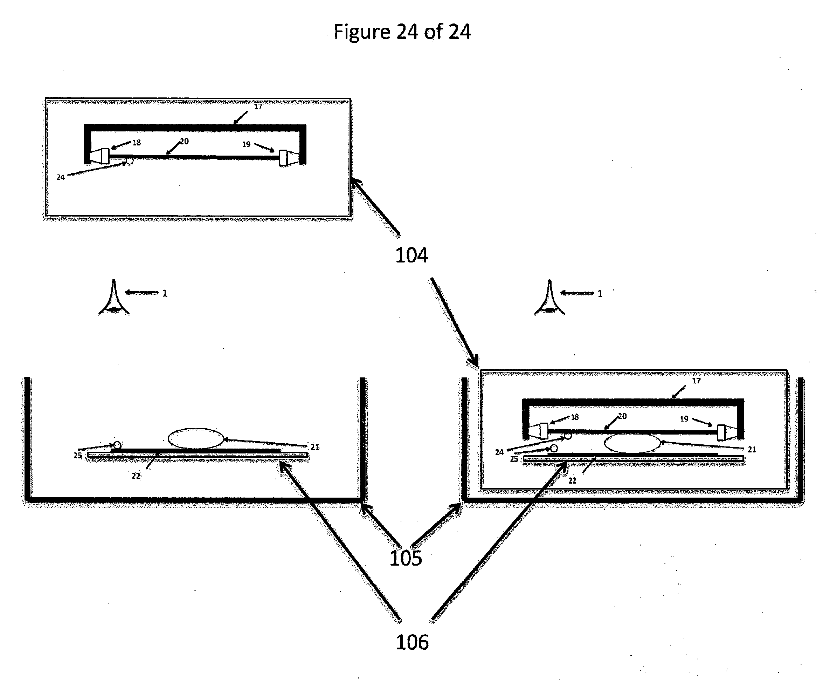

Figure 19 shows a composite heat flux plate that can accommodate crucibles of widely different diameters (see also Figure 20 and 24). It comprises four primitive heat flux plates 90, 93, 94 and 95 assembled to form a large circular composite plate. Each of the four primitive heat flux plates has a circular sample position 91 and an ark-shaped reference position 92.

Figure 20 illustrates how, by making electrical connection 96 between two of the primitive heat flux plates 93 and 95, a single heat flux plate is formed. A sample crucible 97 and reference crucible 98 can be placed as shown in the Figure and the composite plate functions like a conventional heat flux DSC.

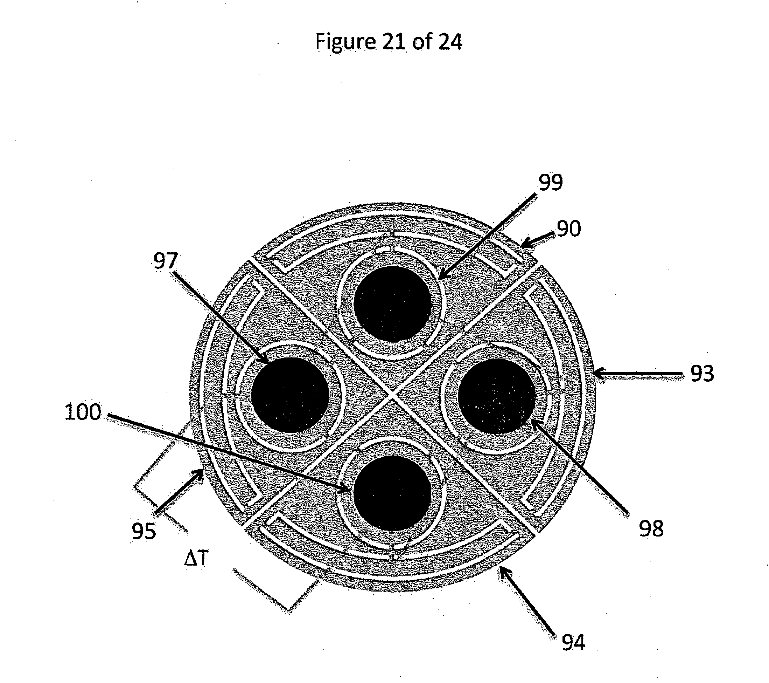

Figure 21 demonstrates how the composite heat flux plate can switch from the conventional mode shown in Figure 20 to sequential connectivity creating a thermopile; the sample position in one primitive heat flux plate is connected to the reference position of the adjacent primitive heat flux plate. These connections are made using a material that forms a thermocouple junction with the material the plates are made of. This configuration offers the option of placing four sample crucibles 97, 98, 99 and 100 on the composite plate. If all of the crucibles contain the same sample this configuration provides a four times improvement in sensitivity (i.e. a X4 increase in the signal for a given transition) compared to the configuration shown in Figure 20, for the same thickness of sample.

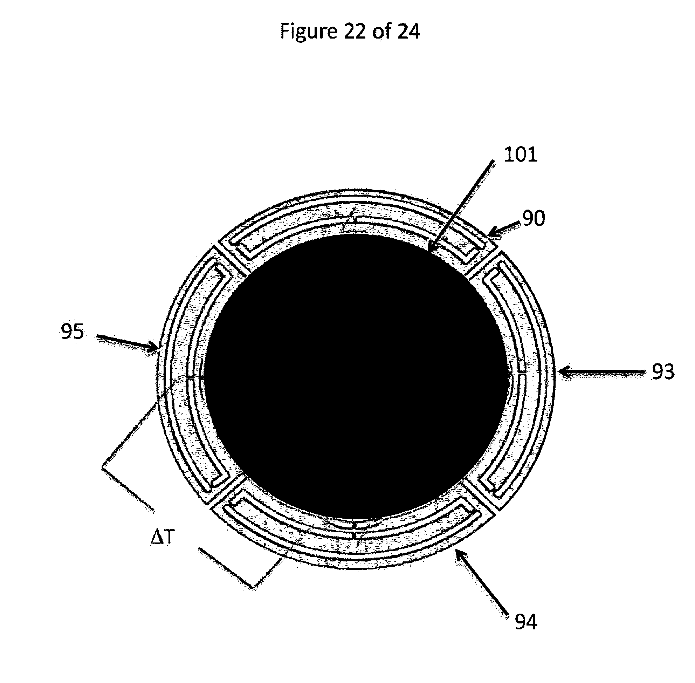

A more useful option than that shown in Figure 21 is shown in Figure 22; a single larger crucible 101 that touches all four of the sample positions shown in Figure 21. With a diameter circa X3 bigger than the sample crucibles shown in Figure 21 the mass of sample would be circa X10 bigger thus providing a X10 improvement in sensitivity (i.e. an X10 increase in the signal for a given transition), for the same thickness of sample.

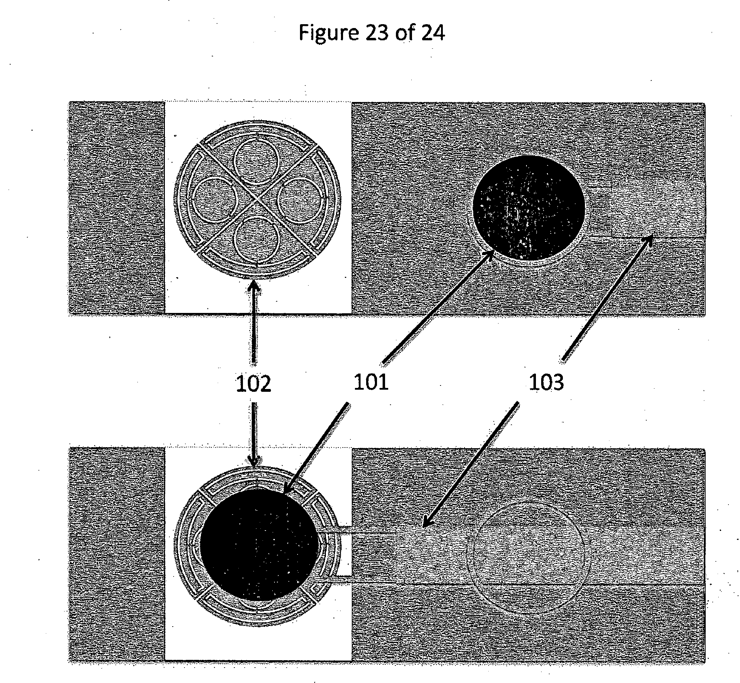

Figure 23 shows that the composite heat flux plate described in Figures 19, 20, 21 and 22, requires a method of locating the different sized crucibles in the right place and in one embodiment of the invention a camera (not shown) is used with suitable software to locate and track the crucible so that, under the action of a force, the crucible 101 is moved with high accuracy to a predetermined place on the composite heat flux plate 102 by actuator 103.

This embodiment of the invention has the following advantages over the conventional method :-
- The sample crucible can be tracked as it enters the oven of the DSC and its position on the heat flux plate can be determined by automated visual inspection.
- It can be used with reference targets to improve the accuracy with which the position of the sample crucible is located.
- The composite heat flux plate can be used with a wide range of sizes of crucible without any change to the device.

Figure 13 can be represented with reference to Figure 1 by designating :-
- Component 2 is an actuator (103).
- Contact component 1 is a crucible (101).
- The sample is the sample (not shown).
- Contact component 2 and components 1, 3, 4, 5, 6, 7 and 8 is a flat plate (69) are not present

J) In sections A) to I) various embodiments of the invention are described, in further embodiments, components are preassembled so that they are capable of being easily inserted into a basic apparatus that comprises the light source or sources, the camera or cameras, the computer and associated control electronics. By inserting the preassembled components then removing them and inserting another preassembled collection of components it is possible to easily switch from one embodiment to another. The preassembled components located within a suitable framework to hold the components in place, is called a Cassette. The use of a Cassette is illustrated in Figure 24 that shows a furnace 105, within which there is an actuator 106 capable of movement in two dimensions, connected to this is contact Object 2 (numbered 22 in the Figure) which is a flat plate. On top of contact Object 2 (numbered 22 in the Figure) there is the sample 21. The cassette 104 contains the components necessary, when united with the components and the sample located within the furnace 105, to form the embodiment illustrated in Figure 5. By extension the embodiments illustrated in Figures 6, 9, 13, 18, and 23 can also be preassembled to form a Cassette.

The embodiments of the invention described in A) to J) above illustrate its versatility. There are many more possible configurations, these include but are not limited to :-
- Configurations that comprise multiples contact points on the sample thus multiple contact components (the maximum given in the examples above is three but this is not the maximum that can be used). For example, multiple contact components could be use to exert forces in different directions in order to evaluated anisotropy.
- Force transducers could be used instead of or as well as springs. For example where a component is fixed it could be replaced by or attached to a force transducer to provide additional information and/or to extend the range of forces that can be measured and/or exerted.
- Force Transducers could be used in association with actuators. For example in Figure 1 components 1,4,5 and 8 could be actuators while component 2,3,6 and 7 could be force transducers while contact components 1a, 1b, 2a and 2b cause the sample to undergoes complex distortions that are characterized by the camera observing the sample as the target. In this way samples with complex shapes could be analyzed.
- More than one camera could be used to achieve stereoscopic vision and so the positions of targets in three-dimensional space could be tracked and/or top and bottom views could be acquired simultaneously.

## Claims

1. An apparatus for the thermo-mechanical characterisation of a sample (4), said apparatus comprising a chamber configured to heat or cool the sample (4) and a set of components (2,3,7,8,9,10,12) located within the chamber, said apparatus including at least one light source and at least one camera situated outside of the chamber, said camera (1) operable to observe one or more of the set of components that are connected to the sample and that apply a force to the sample, **characterised in that** the location and movement of a component or a plurality of components (2,3,7,8,9,10,12) simultaneously is measured and tracked, including tracking motion that is linear or a two dimensional motion, by analyzing the images (16, 35,34) obtained from the camera using suitable software.

2. Apparatus as claimed in claim 1 wherein one or more of the selected components are Targets (13, 14) that are distinctive in appearance thereby making it possible for there to be few errors when locating and tracking the Targets by the camera (1) when used with suitable software.

3. Apparatus as claimed in claims 1 and 2 wherein the background behind the sample (4) and/or the observed components (2,3,7,8,9,10,12) are distinctive in appearance thereby making it possible for there to be few errors when distinguishing between the background and the sample and/or selected components by the camera when used with suitable software.

4. Apparatus as claimed in any preceding claim wherein one or more of the selected components (2,3,7,8,9,10,12) are Targets (13,14) and said Targets are reflective.

5. Apparatus as claimed in any preceding claim wherein one or more of the set of components (2,3,7,8,9,10,12) comprises an actuator (6,23).

6. Apparatus as claimed in any preceding claim wherein one or more of the set of components (2,3,7,8,9,10,1.2) comprises a spring (8,9).

7. Apparatus as claimed in any preceding claim wherein one or more components (2,3,7,8,9,10,12) comprises a force transducer (2,3,6,7).

8. Apparatus as claimed in any preceding claim wherein the set of components (2,3,7,8,9,10,12) is preassembled and said preassembled components are located in a cassette (104).

9. A method of measuring the thermo-mechanical properties of a sample (4) as a function of time and temperature comprising placing a sample and a number of components (2,3,7,8,9,10,12) into a chamber configured to heat or cool the sample and the components, one or more of these components being in contact with the sample (4) and applying a force to the same; illuminating the sample with at least one light source; observing a selected component or a plurality of selected components with at least one camera (1); analyzing the images (16, 35,34) obtained with the camera by means of suitable software thereby measuring the location and tracking the movement of the selected components or the plurality of selected components simultaneously thereby to obtain data in respect of the properties of the sample as a function of time and temperature.

10. Method as claimed in claim 9 comprising the step of selecting one or more components (2,3,7,8,9,10,12) that are Targets that are distinctive in appearance thereby to cause the camera (1) used with suitable software to locate and track these components.

11. Method as claimed in claim 9 or claim 10 comprising the step of selecting a background behind the sample (4) and/or the observed components (2,3,7,8,9,10,12), that is distinctive in appearance thereby to enable the background to be easily distinguished from the sample and/or selected components thereby to enable the camera used with suitable software to locate and track the sample and/or the selected components with few errors.

12. Method as claimed in anyone of claims 9 to 11 comprising the step of causing the sample (4) to move to a selected location and/or to deform using at least one component to apply a force to the sample.

13. Method as claimed in anyone of claims 9 to 12 comprising the step of modulating the intensity of at least one light source such that the modulation is in synchrony with the movement of the sample (4).

14. Method as claimed in anyone of claims 9 to 13 comprising the step of observing an image reflected from a component (2,3,7,8,9,10,12) acting as a mirror.

## Patentansprüche

1. Vorrichtung zur thermomechanischen Charakterisierung einer Probe (4), wobei die genannte Vorrichtung eine Kammer, die zum Erhitzen oder Kühlen der Probe (4) konfiguriert ist, und einen Satz von in der Kammer liegenden Komponenten (2, 3, 7, 8, 9, 10, 12) aufweist, wobei die genannte Vorrichtung wenigstens eine Lichtquelle und wenigstens eine Kamera, die sich außerhalb der Kammer befindet, beinhaltet, wobei die genannte Kamera (1) zum Beobachten von einer oder mehr des Satzes von Komponenten funktionell ist, die mit der Probe verbunden sind und die eine Kraft auf die Probe ausüben, **dadurch gekennzeichnet, dass** die Lage und Bewegung einer Komponente oder mehrerer Komponenten (2, 3, 7, 8, 9, 10, 12) durch Analysieren der von der Kamera aufgenommenen Bilder (16, 35, 34) unter Verwendung geeigneter Software gleichzeitig gemessen und verfolgt werden, einschließlich dem Verfolgen von Bewegung, die eine lineare oder zweidimensionale Bewegung ist, durch Analysieren der von der Kamera unter Verwendung geeigneter Software aufgenommenen Bilder (16, 35, 34).

2. Vorrichtung nach Anspruch 1, wobei eine oder mehr der ausgewählten Komponenten Targets (13, 14) sind, die ein eindeutiges Aussehen haben, wodurch es möglich wird, dass es bei der Ortung und Verfolgung der Targets mit der Kamera (1) bei Verwendung mit geeigneter Software wenig Fehler gibt.

3. Vorrichtung nach Anspruch 1 und 2, wobei der Hintergrund hinter der Probe (4) und/oder den beobachteten Komponenten (2, 3, 7, 8, 9, 10, 12) ein eindeutiges Aussehen hat, wodurch es möglich wird, dass es beim Unterscheiden zwischen dem Hindergrund und der Probe und/oder den ausgewählten Komponenten mit der Kamera bei Verwendung mit geeigneter Software wenig Fehler gibt.

4. Vorrichtung nach einem der vorhergehenden Ansprüche, wobei eine oder mehr der ausgewählten Komponenten (2, 3, 7, 8, 9, 10, 12) Targets (13, 14) sind und die genannten Targets reflektierend sind.

5. Vorrichtung nach einem der vorhergehenden Ansprüche, wobei der eine Satz oder die mehr Sätze von Komponenten (2, 3, 7, 8, 9, 10, 12) eine Stelleinrichtung (6, 23) aufweisen.

6. Vorrichtung nach einem der vorhergehenden Ansprüche, wobei der eine Satz oder die mehr Sätze von Komponenten (2, 3, 7, 8, 9, 10, 12) eine Feder (8, 9) aufweisen.

7. Vorrichtung nach einem der vorhergehenden Ansprüche, wobei eine oder mehr Komponenten (2, 3, 7, 8, 9, 10, 12) einen Kraftaufnehmer (2, 3, 6, 7) umfassen.

8. Vorrichtung nach einem der vorhergehenden Ansprüche, wobei der Satz von Komponenten (2, 3, 7, 8, 9, 10, 12) vormontiert ist und die genannten vormontierten Komponenten sich in einer Kassette (104) befinden.

9. Verfahren zum Messen der thermomechanischen Eigenschaften einer Probe (4) als eine Funktion von Zeit und Temperatur, umfassend Einsetzen eines Musters und einer Anzahl von Komponenten (2, 3, 7, 8, 9, 10, 12) in eine Kammer, die zum Erhitzen oder Kühlen der Probe und der Komponenten konfiguriert ist, wobei eine oder mehr dieser Komponenten mit der Probe (4) in Kontakt sind und eine Kraft auf dieselbe ausüben; Beleuchten der Probe mit wenigstens einer Lichtquelle; Beobachten einer ausgewählten Komponente oder mehrerer ausgewählter Komponenten mit wenigstens einer Kamera (1); Analysieren der mit der Kamera aufgenommenen Bilder (16, 35, 34) mithilfe geeigneter Software, wodurch die Lage und die Bewegung der ausgewählten Komponenten oder der mehreren ausgewählten Komponenten gleichzeitig gemessen bzw. verfolgt wird, um dadurch Daten in Bezug auf die Eigenschaften der Probe als eine Funktion von Zeit und Temperatur zu erhalten.

10. Verfahren nach Anspruch 9, das den Schritt des Auswählens von einer oder mehr Komponenten (2, 3, 7, 8, 9, 10, 12) aufweist, die Targets sind, die ein eindeutiges Aussehen haben, um dadurch die mit geeigneter Software verwendete Kamera (1) zum Orten und Verfolgen dieser Komponenten zu veranlassen.

11. Verfahren nach Anspruch 9 oder Anspruch 10, das den Schritt des Auswählens eines Hintergrunds hinter der Probe (4) und/oder den beobachteten Komponenten (2, 3, 7, 8, 9, 10, 12) aufweist, der ein eindeutiges Aussehen hat, um es dadurch möglich zu machen, dass der Hintergrund von der Probe und/oder ausgewählten Komponenten leicht unterscheidbar ist, um es dadurch der mit geeigneter Software verwendeten Kamera zu ermöglichen, die Probe und/oder die ausgewählten Komponenten mit wenig Fehlern zu orten und zu verfolgen.

12. Verfahren nach einem der Ansprüche 9 bis 11, das den Schritt des Veranlassens der Probe (4) zum Bewegen auf eine ausgewählte Position und/oder zum Verformen unter Verwendung wenigstens einer Komponente zum Ausüben einer Kraft auf die Probe aufweist.

13. Verfahren nach einem der Ansprüche 9 bis 12, das den Schritt des Modulierens der Intensität der wenigstens einen Lichtquelle aufweist, so dass die Modulation mit der Bewegung der Probe (4) synchron erfolgt.

14. Verfahren nach einem der Ansprüche 9 bis 13, das den Schritt des Beobachtens eines von einer als Spiegel wirkenden Komponente (2, 3, 7, 8, 9, 10, 12) reflektierten Bilds aufweist.

## Revendications

1. Appareil pour la caractérisation thermomécanique d'un échantillon (4), ledit appareil comprenant une chambre configurée de façon à chauffer ou à refroidir l'échantillon (4), et un ensemble de composants (2, 3, 7, 8, 9, 10, 12) positionnés à l'intérieur de la chambre, ledit appareil incluant au moins une source lumineuse et au moins une caméra située à l'extérieur de la chambre, ladite caméra (1) étant utilisable pour observer un ou plusieurs composants de l'ensemble de composants qui sont raccordés à l'échantillon et qui appliquent une force à l'échantillon, **caractérisé en ce que** la localisation et le mouvement d'un composant ou d'une pluralité de composants (2, 3, 7, 8, 9, 10, 12) en simultanéité font l'objet d'une mesure et d'un suivi, incluant un mouvement de suivi qui est linéaire ou un mouvement bidimensionnel, grâce à l'analyse des images (16, 35, 34) obtenues à partir de la caméra utilisant un logiciel approprié.

2. Appareil selon la revendication 1, un ou plusieurs des composants sélectionnés étant des Cibles (13, 14) qui sont distinctives de par leur apparence, ce qui permet par conséquent de n'avoir que peu d'erreurs lors de la localisation et du suivi des Cibles par la caméra (1) lorsqu'elle est utilisée avec un logiciel approprié.

3. Appareil selon les revendications 1 et 2, l'arrière-plan derrière l'échantillon (4) et/ou les composants observés (2, 3, 7, 8, 9, 10, 12) étant distinctifs de par leur apparence, ce qui permet par conséquent de n'avoir que peu d'erreurs lors de la distinction entre l'arrière-plan et l'échantillon et/ou les composants sélectionnés par la caméra lorsqu'elle est utilisée avec un logiciel approprié.

4. Appareil selon l'une quelconque des revendications précédentes, un ou plusieurs des composants sélectionnés (2, 3, 7, 8, 9, 10, 12) étant des Cibles (13, 14), et lesdites Cibles étant réfléchissantes.

5. Appareil selon l'une quelconque des revendications précédentes, un ou plusieurs composants de l'ensemble de composants (2, 3, 7, 8, 9, 10, 12) comprenant un actionneur (6, 23).

6. Appareil selon l'une quelconque des revendications précédentes, un ou plusieurs composants de l'ensemble de composants (2, 3, 7, 8, 9, 10, 12) comprenant un ressort (8, 9).

7. Appareil selon l'une quelconque des revendications précédentes, un ou plusieurs composants (2, 3, 7, 8, 9, 10, 12) comprenant un transducteur de force (2, 3, 6, 7).

8. Appareil selon l'une quelconque des revendications précédentes, l'ensemble de composants (2, 3, 7, 8, 9, 10, 12) étant pré-assemblé et lesdits composants pré-assemblés étant localisés dans une cassette (104).

9. Procédé destiné à mesurer les propriétés thermomécaniques d'un échantillon (4) en fonction du temps et de la température, comprenant les opérations consistant à placer un échantillon et un certain nombre de composants (2, 3, 7, 8, 9, 10, 12) dans une chambre configurée de façon à chauffer ou à refroidir l'échantillon et les composants, un ou plusieurs de ces composants étant au contact de l'échantillon (4), et à appliquer une force sur celui-ci ; à éclairer l'échantillon à l'aide d'au moins une source lumineuse ; à observer un composant sélectionné ou une pluralité de composants sélectionnés avec au moins une caméra (1) ; à analyser les images (16, 35, 34) obtenues avec la caméra au moyen d'un logiciel approprié, ce qui permet par conséquent de mesurer la localisation et d'assurer le suivi du mouvement des composants sélectionnés ou de la pluralité de composants sélectionnés, en simultanéité, permettant ainsi d'obtenir des données au sujet des propriétés de l'échantillon en fonction du temps et de la température.

10. Procédé selon la revendication 9, comprenant l'étape consistant à sélectionner un ou plusieurs composants (2, 3, 7, 8, 9, 10, 12) qui sont des Cibles lesquelles sont distinctives de leur apparence, ce qui oblige par conséquent la caméra (1) utilisée avec un logiciel approprié à assurer la localisation et le suivi de ces composants.

11. Procédé selon la revendication 9 ou la revendication 10, comprenant l'étape consistant à sélectionner un arrière-plan derrière l'échantillon (4) et/ou les composants observés (2, 3, 7, 8, 9, 10, 12) lesquels sont distinctifs de par leur apparence, ce qui permet par conséquent de faire facilement la distinction entre l'arrière-plan et l'échantillon et/ou les composants sélectionnés et permet ainsi à la caméra utilisée avec un logiciel approprié d'assurer, avec peu d'erreurs, la localisation et le suivi de l'échantillon et/ou des composants sélectionnés.

12. Procédé selon l'une quelconque des revendications 9 à 11, comprenant l'étape consistant à obliger l'échantillon (4) à se déplacer vers une localisation sélectionnée et/ou provoquer sa déformation grâce à l'utilisation d'au moins un composant pour appliquer une force à l'échantillon.

13. Procédé selon l'une quelconque des revendications 9 à 12, comprenant l'étape consistant à moduler l'intensité de ladite au moins une source lumineuse pour que la modulation soit en synchronisation avec le mouvement de l'échantillon (4).

14. Procédé selon l'une quelconque des revendications 9 à 13, comprenant l'étape consistant à observer une image réfléchie à partir d'un composant (2, 3, 7, 8, 9, 10, 12) qui joue le rôle de miroir.
